# EUROPÄISCHE PATENTSCHRIFT

(11) **EP 0 577 996 B1**
(45) Veröffentlichungstag und Bekanntmachung des Hinweises auf die Patenterteilung: **09.10.1996**
(21) Anmeldenummer: 93109388.4
(22) Anmeldetag: 11.06.1993
(51) Int. Cl.: A61K 35/50, A61K 7/48

(54) **Placentaextrakt und Verfahren zu seiner Herstellung**
Placenta extract and process for preparing it
Extrait placentaire et son procédé d'obtention

(30) Priorität: 11.06.1992 CH 1855/92
(43) Veröffentlichungstag der Anmeldung: 12.01.1994
(73) Patentinhaber: Laboratoire Lucchini S.A., CH-1204 Genève (CH)
(72) Erfinder: Martin, Michel Pierre, CH-1206 Genève (CH); De Schoenburg Waldenburg, Vetea Pierre, F-01220 Divonne-Les-Bains (FR)
(74) Vertreter: Zink-Wild, Markus Peter

(56) Entgegenhaltungen:
- AT-A- 366 577
- US-A- 3 504 084
- DATABASE WPI Section Ch, Week 7338, Derwent Publications Ltd., London, GB; Class B04, AN 73-56444U
- 'DICTIONNAIRE VIDAL, EDITION 1991' 1991 , EDITIONS DU VIDAL , PARIS, FR, mittlere Spalte, "Placentafil".
- W.P. Filatow, MMW, Nr. 31, 1955, S. 1016-1019

## Beschreibung

Die vorliegende Erfindung betrifft einen Extrakt und ein Verfahren zur Herstellung dieses Extraktes.

Placentas sind schon auf die unterschiedlichsten Arten extrahiert worden. Verschiedene Produkte auf der Basis solcher Extrakte sind bekannt. Der nächste Stand der Technik ist beschrieben in:
BURT R.L. et. al. (1966) Am. J. Obst. and Gyn. St-Louis, 95, 579 - 585;
RAUCH S. (1956) JAMA, 162, 915;
GOLD-AUBERT P. et. al. (1981) Int. J. Tiss. Reac. III, 155 - 165; und
NAKAJIMA S. (1971) J. Med. Soty Toho Uni. 18, 368 - 372 "English translation".

Völlig überraschend wurde nun ein Extrakt gefunden, welcher eine therapeutische und/oder eine biologische Aktivität zeigt. Seine exakte Zusammensetzung ist gegenwärtig noch nicht bekannt. Dieser Extrakt kann nur mittels den erfindungsgemässen Verfahrensschritten gewonnen werden. Es ist durchaus denkbar, dass der eine oder der andere Schritt weggelassen werden kann, ohne dass dies einen Einfluss auf die genannte biologische und/oder therapeutische Aktivität hat; z.B. die Schritte A, B und C.

Die Erfindung ist durch die Merkmale in den unabhängigen Ansprüchen gekennzeichnet. Bevorzugte Ausführungsformen sind in den abhängigen Ansprüchen definiert.

Im Folgenden wird die Erfindung näher erläutert.

Die zu extrahierenden Placentas können menschlichen wie auch tierischen Ursprungs sein. Im Prinzip könnten die Placentas unmittelbar nach einer Geburt oder Abtrennung verarbeitet werden. Dies ist jedoch wegen Transportengpässen nur begrenzt möglich. Aus praktischen Gründen werden die menschlichen Placentas in den Spitälern in Behältern in Tiefkühlboxen gelagert, wobei darauf geachtet wird, dass im Tiefkühlgerät die Temperatur nicht höher als -10°C ist. In Abhängigkeit des jeweils verwendeten Tiefkühlgerätes beträgt die Lagerungs-Temperatur normalerweise von etwa -18°C bis etwa -25°C. Noch tiefere Temperaturen sind auch möglich, sind aber nicht wirtschaftlich.

Menschliche Placentas ohne Risikofaktoren sind bevorzugt.

Es ist bevorzugt, die Placentas zu verarbeiten, bevor sie 3 bis 4 Monate alt sind.

Zur Herstellung des erfindungsgemässen Extraktes werden die tiefgefrorenen Placentas unter sterilen Bedingungen (UV-Licht) auf Raumtemperatur aufwärmen gelassen. In Abhängigkeit der Menge an gefrorenen Placentas kann dies bis zu 2 Tagen dauern. Vorzugsweise werden die Placentas in Kunststoffbehältern (plastic vats) in einem Schrank (cup-board) aufgetaut. Es ist bevorzugt, unter hygienisch kontrollierten Bedingungen zu arbeiten. So ist nicht kontaminiertes Material zu verwenden, und die Arbeitsräume sind sauber zu halten.

Die Placentas können auch durch Einwirkung von Wärme und/oder Mikrowellen aufgetaut werden.

Von jeder aufgetauten Placenta wird nun die Chorion, auch Zottenhaut genannt [la coiffe (chorion)], die Nabelschnur [le cordon (cord)], Blutgerinnsel und nekrotisches Gewebe entfernt. Vorzugsweise werden diese Teile manuell mit einem Messer oder Skalpell weggeschnitten. Vom so erhaltenen Gewebe der Placenta wird nun unter Einwirkung von Druck Blut entfernt. Dies wird vorzugsweise mittels manuellem Zusammendrücken oder Zentrifugation durchgeführt.

Die so erhaltenen Placenta-Gewebe werden nun abtropfen gelassen und gegebenenfalls getrocknet. Das Trocknen kann zum Beispiel mit einem Schwamm, einem Tuch oder mittels Papier erfolgen, wobei Wasser und gegebenenfalls noch vorhandenes Blut entfernt werden.

Die so abgetropften und getrockneten Placenta-Gewebe werden nun wieder eingefroren, wobei wieder darauf geachtet wird, dass im Tiefkühlgerät die Temperatur nicht höher als -10°C ist. In Abhängigkeit des jeweils verwendeten Tiefkühlgerätes beträgt die Lagerungs-Temperatur normalerweise von etwa -18°C bis etwa -25°C. Noch tiefere Temperaturen sind auch möglich, sind aber nicht wirtschaftlich. Eine Schockeinfrierung mit beispielsweise flüssigem Stickstoff ist auch möglich.

An dieser Stelle hat man die Möglichkeit, das Verfahren für einige Tage oder Monate, z.B. 3 bis 4 Monate, zu unterbrechen.

Die eingefrorenen Placenta-Teile werden im nächsten Arbeitsschritt im gefrorenen Zustand zerkleinert. Sie werden insbesondere zu einem Brei zerhackt, resp. püriert. Hierin sollen keine Teilchen mit einem Durchmesser von mehr als 5 mm, vorzugsweise 3 mm, vorhanden sein. Eine Zerkleinerung in nicht gefrorenem Zustand wäre auch möglich.

Diese breiige Masse wird nun mit kaltem Wasser vermischt. Dieses kalte Wasser hat normalerweise eine Temperatur von etwa + 1°C bis etwa + 10°C, vorzugsweise von etwa + 4°C bis etwa + 10°C. Destilliertes, d.h. ionenfreies Wasser ist bevorzugt.

Die so erhaltene Suspension wird anschliessend während wenigstens 15 Minuten mechanisch gerührt. Eine zusätzliche Behandlung mit Ultraschall ist auch möglich.

Danach lässt man die Suspension bei einer Temperatur von etwa + 4°C, insbesondere während etwa 1 Stunde, in einem Kühlschrank oder einem Kühlraum, stehen. In Abhängigkeit des jeweils verwendeten Kühlschrankes oder Kühlraumes kann die Temperatur auch etwas höher oder etwas tiefer sein, beispielsweise ± 1 bis 2°C.

Dann wird die gekühlte Suspension aus dem Kühlraum entfernt, und bei umgebender Temperatur wird unter mechanischem Rühren festes Natriumchlorid in einer solchen Menge zugegeben, dass eine physiologische Konzentration, d.h. 8,5 g bis 9,0 g NaCl pro kg H₂O, erhalten wird.

Gegebenenfalls kann auch noch eine wässrige, organometallische Lösung hinzugegeben werden, vorzugsweise eine 0,1 %-ige Lösung, beispielsweise eine Kupferacetatlösung, Cu(OAc)₂, insbesondere in einer solchen Menge, dass eine solche Konzentration erhalten wird, welche einer Menge von 1,52 g Cu(OAc)₂ pro kg H₂O entspricht.

Die mit Natriumchlorid, und gegebenenfalls, z.B. auch mit Kupferacetat, versetzte, immer noch kühle Suspension wird nun bei umgebender Temperatur während einigen Minuten oder Stunden, beispielsweise etwa 30 Minuten, mechanisch gerührt. Eine zusätzliche Behandlung mit Ultraschall ist auch möglich. Bei diesem Rühren ist darauf zu achten, dass die Temperatur + 15°C, insbesondere + 10°C, nicht überschreitet. Gegebenenfalls muss die Suspension gekühlt werden, damit die genannte Temperatur eingehalten werden kann.

Danach lässt man diese Suspension während etwa einigen Stunden, beispielsweise etwa 4 Stunden, wieder bei einer Temperatur von etwa + 4°C in einem Kühlschrank oder einem Kühlraum stehen. In Abhängigkeit des jeweils verwendeten Kühlschrankes oder Kühlraumes kann die Temperatur auch etwas höher oder etwas tiefer sein, beispielsweise ± 1 bis 2°C.

Dann wird die gekühlte Suspension aus dem Kühlraum entfernt, und bei umgebender Temperatur wird unter mechanischem Rühren der pH-Wert gemessen. Im allgemeinen liegt dieser pH-Wert im Bereich zwischen etwa 6,6 und etwa 6,9. Diese Suspension wird anschliessend angesäuert, wobei ein pH-Wert von etwa 5,3 bevorzugt ist. Das Ansäuern wird vorzugsweise durch Hinzugabe von Milchsäure durchgeführt.

Danach lässt man die angesäuerte Suspension während einigen Stunden, z.B. 15 Stunden, bei einer Temperatur von etwa + 4°C in einem Kühlschrank oder einem Kühlraum stehen. Betreffend einer Temperaturabweichung wird auf die oben stehenden Ausführungen verwiesen.

An dieser Stelle hat man wieder die Möglichkeit, das Extraktionsverfahren für einige Tage zu unterbrechen.

Anschliessend wird die Suspension zentrifugiert, und die überstehende Lösung wird gesammelt. Diese Lösung wird filtriert, um ein klares Filtrat zu erhalten. Diese Filtration kann mit einem oder mehreren Filtern gleicher oder unterschiedlicher Porosität erfolgen. Ebenso kann diese Filtration unter Druck oder mittels Vakuum durchgeführt werden.

Das so erhaltene Filtrat hat normalerweise einen pH-Wert von etwa 5,7 bis etwa 6,1. Dieses Filtrat wird angesäuert, wobei ein pH-Wert von etwa 5,4 bevorzugt ist. Das Ansäuern wird vorzugsweise durch Hinzugabe von Milchsäure durchgeführt. Bei diesem Ansäuern bilden sich gewöhnlich Niederschläge, welche mittels erneuter Filtration durch einen oder mehrere Filter gleicher oder unterschiedlicher Porosität entfernt werden. Diese Filtration kann unter Druck oder mittels Vakuum durchgeführt werden.

Das so erhaltene Filtrat kann nun mittels Filtration sterilisiert werden. Es versteht sich von selbst, dass sämtliche dazu benötigten Gegenstände vorgängig sterilisiert werden müssen, beispielsweise in einem Dampfsterilisator bei einer Temperatur von 127°C während 30 Minuten. Ebenso ist klar, dass diese Sterilisationsfiltration unter aseptischen Bedingungen in sterilen Räumen durchzuführen ist.

Das erhaltene, vorzugsweise sterile Filtrat kann - in geeignete Behälter abgefüllt - nun inkubiert werden. Diese Inkubation erfolgt vorzugsweise bei einer Temperatur von etwa + 35°C bis etwa + 45°C, insbesondere etwa + 39°C, während 4 bis 6 Tagen. Proben können jederzeit entnommen werden und einem Sterilitätstest, beispielsweise auf Thioglycolat Medium, unterworfen werden.

Die gegebenenfalls inkubierte Lösung wird anschliessend pasteurisiert. Diese Pasteurisierung erfolgt vorzugsweise bei einer Temperatur von etwa + 60°C während wenigstens 14 Stunden. Man erhält eine nicht mehr klare Lösung. Die entstandenen Niederschläge werden mittels oben beschriebener Zentrifugation und gegebenenfalls einer sterilen Filtration entfernt.

Dieses Filtrat kann nun, eingestellt auf eine geeignete Konzentration, wenigstens einer üblichen Ultrafiltration ausgesetzt werden, um Verbindungen mit vorausbestimmten Molekulargewichten entweder zu entfernen oder zu erhalten.

So kann das genannte Filtrat einer Ultrafiltration - nachfolgend Ultrafiltration I genannt - ausgesetzt werden, um Verbindungen mit Molekulargewichten von kleiner als 30'000 zu entfernen. Diese entfernten Verbindungen werden separat gesammelt.

Die erhaltene Lösung kann nun angesäuert werden, beispielsweise bis zu einem pH-Wert von 1,0, vorzugsweise mit Salzsäure, insbesondere mit wässriger Salzsäure.

Die angesäuerte Lösung wird während einigen Stunden, beispielsweise 15 bis 24 Stunden, inkubiert.

In der so erhaltenen Lösung wird ein gemessener pH-Wert im Bereich von 5,0 bis 8,0, vorzugsweise 7,0, eingestellt.

Dieses Gemisch kann danach, eingestellt auf eine geeignete Konzentration, einer weiteren üblichen Ultrafiltration - nachfolgend Ultrafiltration II genannt - ausgesetzt werden, um Verbindungen mit Molekulargewichten von grösser als 300'000 zu entfernen. Diese entfernten Verbindungen werden separat gesammelt.

Es ist auch möglich, zuerst die Ultrafiltration II und dann die Ultrafiltration I auszuführen.

Die Reihenfolge, in welcher die Ultrafiltrationen I und II sowie die pH-Einstellung auf einen sauren Wert ausgeführt werden, hat keinen Einfluss auf das Endprodukt.

Es ist auch möglich, eine übliche Ultrafiltration auszuführen, um Verbindungen mit Molekulargewichten von grösser als 300'000 zu entfernen und separat zu sammeln. Das erhaltene Filtrat kann einer zweiten üblichen Ultrafiltration (100 K-Membrane) ausgesetzt werden.

Auf der Membrane bleibt eine konzentrierte Lösung von Verbindungen mit Molekulargewichten von 100'000 bis 300'000 zurück. Das so erhaltene Filtrat kann einer dritten üblichen Ultrafiltration (5 K-Membrane) ausgesetzt werden, um Verbindungen mit Molekulargewichten von 5'000 bis 100'000 zu entfernen und separat zu sammeln.

Als letzte Operation kann man in vorausbestimmten Verhältnissen das zuletzt erhaltene Filtrat (0-5000) mit dem Rückstand der zweiten Ultrafiltration (100'000 - 300'000) vermischt werden, um ein ausgewähltes Produkt zu ergeben.

Alle mittels Ultrafiltrationen entfernten Verbindungen können separat gesammelt und für andere Zwecke verwendet werden.

An Stelle der genannten Ultrafiltrationen können auch Gel-Filtrationen durchgeführt werden.

Die auf diese Weise - und völlig überraschend nur auf diese Weise - erhaltenen erfindungsgemässen Extrakte zeigen biologische und/oder therapeutische Aktivitäten. Wenn gewünscht, können diese Extrakte gefriergetrocknet werden. Diese erfindungsgemässen Extrakte können verwendet werden zur Herstellung von Mitteln, welche biologische und/oder therapeutische Aktivitäten aufweisen.

Die so erhaltenen Extrakte können als wenigstens eine aktive Komponente in einem Medikament, insbesondere für die Wiederepithelisierung oder für die Behandlung von Neuropathien, verwendet werden.

Die nachfolgenden Beispiele illustrieren die Erfindung.

### Beispiel 1

Von aufgetauten menschlichen Placentas wurden die Chorion, die Nabelschnur, die Blutgerinnsel und nekrotischen Gewebe mit einem Messer entfernt. Vom so erhaltenen Placenta-Gewebe wurde mittels manuellem Zusammendrücken Blut entfernt. Diese Placenta-Gewebe liess man abtropfen und trocknete sie dann mit Papier ab. Danach wurden die Placenta-Gewebe in einem Tiefkühlgerät bei -18°C eingefroren.

Im Folgenden werden 2 voneinander getrennt durchgeführte Ansätze beschrieben. Zahlenangaben, welche den zweiten Ansatz betreffen, sind in Klammern erwähnt.

32,2 kg (33,6 kg) Placenta-Gewebe wurden in gefrorenem Zustand in einer Mühle zerhackt. Zu dieser breiigen Masse wurden 193 kg (193 kg) Wasser mit einer Temperatur von + 4° C hinzugegeben. Diese Suspension wurde sofort während 30 Minuten bei Raumtemperatur mechanisch gerührt. Zum Rühren wurde ein POLYTRON kinematisches Hochfrequenz-Rührgerät verwendet. Normalerweise wurde eine Geschwindigkeit von 2700 bis 3000 Umdrehungen pro Minute verwendet. Danach wurde das Gemisch in einem Kühlraum, in welchem die Temperatur + 4° C betrug, während einer Stunde stehen gelassen. Bei umgebender Temperatur wurden unter mechanischem Rühren 1,640 kg (1,640 kg) festes Natriumchlorid und 292 ml (292 ml) 0,1 %-ige wässrige Cu(OAc)₂-Lösung hinzugegeben. Das mit Natriumchlorid und Kupferacetat versetzte Gemisch wurde sofort während 30 Minuten gerührt. Es wurde darauf geachtet, dass die Temperatur der Suspension + 10°C nicht überstieg. Danach liess man diese Suspension während 4 Stunden in einem Kühlraum, in welchem die Temperatur + 4°C betrug, stehen. Nach dieser Zeit wurde ein pH-Wert von 6,80 (6,85) gemessen. Durch Hinzugabe von 220 ml (173 ml) Milchsäure (Reinheit: 88-92 %) wurde ein pH-Wert von 5,3 (5,3) eingestellt.

Die angesäuerte Suspension wurde über Nacht in einem Kühlraum bei einer Temperatur von + 4°C gelagert. Am nächsten Tage wurde die Suspension zentrifugiert, und die überstehende Lösung wurde gesammelt und filtriert. Man erhielt 183 kg (188 kg) an klarem Filtrat, welches einen pH-Wert von 5,70 (5,70) hatte. Durch erneute Hinzugabe von 20 ml (28 ml) Milchsäure (Reinheit : 88-92 %) wurde ein pH-Wert von 5,4 (5,4) eingestellt. Da sich bei diesem Ansäuern wieder ein Niederschlag bildete, wurde dieser Niederschlag durch eine weitere Filtration entfernt.

Dieses Filtrat wurde anschliessend mittels Filtration sterilisiert ("Fibra Fix"-Filter AF 130 und AF 140 von Filtrox-Werk AG, St. Gallen, Schweiz und Filterkapsel SUPOR DFC 0,2 Mikrometer Gelmann Science von Skan AG, Basel, Schweiz). Dieses sterile Filtrat wurde in 10-Literflaschen abgefüllt, und diese Flaschen wurden bei einer Temperatur von + 39°C während 142 Stunden und 20 Minuten (144 Stunden und 15 Minuten) inkubiert.

Danach wurde eine Pasteurisierung bei einer Temperatur von + 60°C während 27 Stunden (27 Stunden) durchgeführt. Bei dieser Pasteurisierung wurde darauf geachtet, dass die Temperatur des Gemisches in den Flaschen während wenigstens 14 Stunden + 60°C betrug.

Nach der Pasteurisation wurde das Gemisch während 6 Tagen (5 Tagen) in einem Kühlraum bei einer Temperatur von + 4°C gelagert.

Die pasteurisierte, nicht mehr klare Lösung wurde zentrifugiert, und die überstehenden Lösungen von beiden Ansätzen wurden gesammelt und vereint. Die Lösung wurde mittels Filtration sterilisiert, wobei man insgesamt 360 kg an Filtrat erhielt (Ende Schritt N).

### Beispiel 2

Die therapeutische Aktivität des gemäss Beispiel 1 erhaltenen Filtrates wird durch eine beschleunigte Wundheilung belegt.

Männliche New-Zealand Kaninchen wurden einzeln in einem Käfig bei einem konstanten hell/dunkel-Zyklus und bei Raumtemperatur (21°C) gehalten.

In beiden Augen der Kaninchen wurden korneale Ulzerationen durch Einschnitte in die Kornea mit Hilfe eines Glaszylinders (Durchmesser: 3 mm) gemacht. Der Einschnitt wurde oberflächlich in die optische Zone der Kornea gemacht, wobei das Stroma geschont wurde. Eine erste Prüfung unter einer Spaltlampe diente dazu, nur Tiere mit kornealen Ulzerationen des gleichen Ausmasses und Tiefe zu verwenden.

Vorgängig der Behandlung und dann alle 12 Stunden während der Behandlung wurden die Tiere einer ophthalmologischen Prüfung der Korneas mittels der Spaltlampe unterworfen. Vor jeder Prüfung wurde eine Instillation mit 1 %-igem Natriumfluorescein gemacht. Für diese Prüfungen wurde kobaltblaues Licht verwendet. Es wurde die totale Heilung der kornealen Ulzerationen abgewartet.

Eine Gruppe von 6 Kaninchen wurde mit einer Instillation des gemäss Beispiel 1 hergestellten Filtrates behandelt. Dazu wurden alle 6 Stunden 2 Tropfen in die Augen gegeben. Diese Behandlung wurde unmittelbar nach der Verletzung mit dem Glaszylinder und der oben erwähnten ersten Prüfung mit der Spaltlampe begonnen.

Eine Gruppe von 8 Kaninchen wurde in der selben Art mit Placebos (physiologische, wässrige Natriumchloridlösung) behandelt, und diente zur Kontrolle. Der Behandlungseffekt wurde evaluiert durch Vergleich der Zeit bis zur vollständigen Heilung der kornealen Ulzerationen zwischen den beiden oben genannten Gruppen. Es wurden folgende Resultate erhalten:

| Prüfung nach (Stunden) | prozentuale Heilung | |
|---|---|---|
| | mit Placebo | mit Placenta-Extrakt |
| 0 | 0 | 0 |
| 12 | 0 | 17 |
| 24 | 0 | 17 |
| 36 | 13 | 67 |
| 48 | 25 | 83 |
| 60 | 50 | 83 |
| 72 | 63 | 100 |
| 84 | 88 | 100 |
| 96 | 100 | 100 |

Diese Zahlen zeigen, dass die Heilung bei der Behandlung mit Placenta-Extrakt früher einsetzt, rascher vor sich geht und früher beendet ist.

### Beispiel 3

Das gemäss Beispiel 1 erhaltene Filtrat (360 kg) wurde einer Ultrafiltration (30 K-Membrane, Typ "Omega", NMWL von Filtron Technology Co. USA) ausgesetzt, um Verbindungen mit Molekulargewichten von kleiner als 30'000 zu entfernen. Etwa 95 Gew.-% des genannten Filtrates passierten die 30 K-Membrane und wurden separat gesammelt. Der Rückstand auf der 30 K-Membrane wurde 12 mal mit je 10 Litern von physiologischer Kochsalzlösung gewaschen.

Der gewaschene Rückstand wurde zwecks optimaler Ausbeute (Waschen der Ultrafiltrationsvorrichtung) mit etwa 20 Litern von physiologischer Kochsalzlösung verdünnt.

Die erhaltene Lösung (etwa 36 kg) wurde mit 72 kg sterilem, destillierten Wasser verdünnt.

Diese Lösung wurde einer Ultrafiltration (300 K-Membrane, Typ "Open Channel" von Filtron Technology Co. USA) ausgesetzt, um Verbindungen mit Molekulargewichten von grösser als 300'000 zu entfernen. Etwa 98 bis 99 Gew.-% dieser Lösung passierten die 300 K-Membrane und wurden separat gesammelt. Der Rückstand auf der 300 K-Membrane wurde zwecks optimaler Ausbeute 4 mal mit je 10 Litern von physiologischer Kochsalzlösung gewaschen.

Das so verdünnte Filtrat (etwa 144 kg) wurde mittels Filtration sterilisiert ("Fibra Fix"-Filter AF 130 und AF 140 von Filtrox-Werk AG, St. Gallen, Schweiz und Filterkapsel SUPOR DFC 0,2 Mikrometer Gelmann Science von Skan AG, Basel, Schweiz).

Im sterilen Filtrat wurde durch Hinzugabe von 25 %-iger wässriger HCl-Lösung ein pH-Wert von 1,0 eingestellt. Die angesäuerte Lösung wurde bei einer Temperatur von +4°C während 15 Stunden aufbewahrt.

Danach wurde mittels Hinzugabe von 5N NaOH ein pH-Wert von 7,05 eingestellt.

Diese neutralisierte Lösung wurde wie oben beschrieben mittels Filtration sterilisiert (Ende Schritt P).

In diesem sterilen Filtrat (Fraktion 30'000 bis 300'000) wurden folgende Werte an Proteinen und NaCl durch Hinzugabe von NaCl und sterilem destillierten Wasser eingestellt:
1 mg Proteine (total) / 1 ml Lösung
8,5 mg NaCl / 1 ml Lösung.
Ausbeute: 260 bis 300 kg Endlösung.

### Beispiel 4

Die therapeutische Aktivität des Filtrates gemäss Beispiel 3 wurde gemäss der in Beispiel 2 beschriebenen Technik belegt.

Man erhielt folgende Resultate:

| Prüfung nach (Stunden) | prozentuale Heilung | |
|---|---|---|
| | mit Placebo | mit Placenta-Extrakt |
| 0 | 0 | 0 |
| 4 | 0 | 0 |
| 8 | 0 | 0 |
| 12 | 0 | 10 |
| 16 | 10 | 20 |
| 20 | 40 | 40 |
| 24 | 50 | 80 |
| 28 | 50 | 90 |
| 32 | 60 | 100 |
| 36 | 70 | |
| 44 | 80 | |
| 52 | 90 | |

Diese Zahlen zeigen, dass die Heilung bei der Behandlung mit Placenta-Extrakt früher einsetzt, rascher vor sich geht und früher beendet ist.

### Beispiel 5

Mit dem gemäss Beispiel 3 erhaltenen Filtrat (Fraktion 30'000 bis 300'000) wurde das peripherische Nervenwachstum geprüft.

Für diesen Zweck wurden 4 Gruppen von je 10 männlichen Sprague-Dawley-Ratten (180 bis 200 g) unter Standard-Bedingungen gehalten (12 Stunden Tag, 12 Stunden Nacht).

Wasser und Nahrung standen in genügenden Mengen jederzeit zur Verfügung.

Die Tiere der Gruppen 1 und 2 erhielten eine einzige intravenöse Injektion an Streptozotocin (100 mg/kg Körpergewicht). Nach einer Woche wurden die Blutglucose-Werte jeden Tag gemessen. Wenn der Glucose-Wert mehr als 150 mg/ml Blut während 3 aufeinanderfolgenden Tagen betrug wurden die Ratten als diabetisch bezeichnet.

Die Tiere der Gruppen 3 und 4 wurden wie folgt operiert: Unter Anästhesie (Ether) wurde der rechte sciatische Nerv durch Zerschneiden der Muskeln freigelegt und mit einer hämostatischen Klemme (haemostatic forceps) während 30 Sekunden zusammengequetscht (crushed), und zwar in einer Entfernung von 6 mm distal vom sciatischen Knotenpunkt (notch). Anschliessend wurde die Wunde wieder geschlossen.

Die Tiere der Gruppen 1 und 3 wurden während 3 Wochen subkutan mit dem gemäss Beispiel 3 erhaltenen Filtrat (Fraktion 30'000 bis 300'000) behandelt. (0,5 mg Protein pro kg Körpergewicht pro Tag).

Die Tiere der Gruppen 2 und 4 wurden gleichzeitig mit dem gleichen Volumen an physiologischer Kochsalzlösung behandelt.

Während der ganzen Behandlung wurde die wiedererlangung von Schmerzempfindung (recovery of sensation) mit dem "Foot-Flick-Test" gegenüber der einen Schmerz verursachenden Wärmeeinwirkung geprüft.

Die Ratten wurden von Hand in ihrer Bewegungsfreiheit eingeschränkt (immobilized). Die rechte Hinterpfote (hindpaw) wurde gegen einen konstant warmen (47° C) Luftstrom in einer Distanz von 2 mm gehalten. Die Zeit, welche benötigt wurde, um die genannte Pfote zurückzuziehen, wurde gemessen.

Es ist bekannt, dass Kontrolltiere die Pfote in weniger als 1,5 Sekunden zurückziehen.

Wenn die getesteten Tiere ihre Pfote in weniger als 3 Sekunden zurückziehen, dann wird die Wiedererlangung von Schmerzempfindung als bestätigt betrachtet.

Wenn die getesteten Tiere ihre Pfote nach 3 Sekunden noch nicht zurückgezogen haben, so wird keine Wiedererlangung von Schmerzempfindung festgestellt.

Der erste Tag von Wiedererlangung von sensomotorischer Funktion wurde für jede Rate einzeln bestimmt.

In der nachfolgenden Tabelle ist der Durchschnittswert der jeweiligen Gruppe angegeben.

| Experimentelle Gruppe | Wiedererlangung von Schmerzempfindung (Tage) |
|---|---|
| 1. Diabetisch + Extrakt | 9,5 plus/minus 0,3 |
| 2. Diabetisch + Kochsalz | 16,5 plus/minus 0,8 |
| 3. Verletzt + Extrakt | 11,3 plus/minus 0,4 |
| 4. Verletzt + Kochsalz | 18,9 plus/minus 0,3 |

Diese Zahlen zeigen, dass die mit der Fraktion 30'000 bis 300'000 behandelten Tiere viel rascher wieder ihre Schmerzempfindung erlangen und somit der genannte Extrakt (Fraktion 30'000 bis 300'000) eine therapeutische Wirkung hat.

## Patentansprüche (Patentansprüche für folgende(n) Vertragsstaat(en): AT, BE, CH, DE, DK, FR, GB, GR, IT, LI, LU, NL, PT, SE, IE)

1. Extrakt mit biologischer und/oder therapeutischer Aktivität, welcher in einem pharmazeutischen und/oder kosmetischen Produkt verwendet werden kann, dadurch erhältlich, dass man die folgenden Schritte ausführt:
- A. Entfernung der Chorion, auch Zottenhaut genannt, der Nabelschnur, Blutgerinnseln und nekrotischem Gewebe von Placentas menschlichen oder tierischen Ursprungs;
- B. Entfernung von Blut von den so erhaltenen Placenta-Geweben unter Einwirkung von Druck, insbesondere durch manuelles Zusammendrücken;
- C. Abtropfen, und gegebenenfalls Abtrocknen, sowie Einfrieren auf eine Temperatur von wenigstens -10°C der so erhaltenen Placenta-Teile;
- D. Zerkleinerung, resp. Pürieren der eingefrorenen Placenta-Teile im gefrorenen Zustand, wobei insbesondere keine Teilchen mit einem Durchmesser von mehr als 5 mm, vorzugsweise 3 mm, vorhanden sind;
- E. Vermischen der so erhaltenen breiigen Masse mit kaltem Wasser, welches gegebenenfalls NaCl enthalten kann;
- F. Rühren der so erhaltenen Suspension während wenigstens 15 Minuten und anschliessendes Stehenlassen bei einer Temperatur von etwa +4°C, beispielsweise während etwa 1 Stunde;
- G. Zugabe von festem Natriumchlorid, um eine physiologische Konzentration, d.h. 8,5 g bis 9,0 g NaCl pro kg H₂O, zu erhalten;
- H. Rühren der erhaltenen Suspension bei einer Temperatur von nicht mehr als + 15°C, insbesondere + 10°C, während einigen Minuten, vorzugsweise etwa 30 Minuten, und anschliessendes Stehenlassen bei einer Temperatur von etwa + 4°C während einigen Stunden, beispielsweise etwa 4 Stunden;
- I. Messen des pH-Wertes und Ansäuern der Suspension, insbesondere auf einen pH-Wert von etwa 5,3, vorzugsweise durch Hinzugabe von Milchsäure;
- J. Lagerung der angesäuerten Suspension während einigen Stunden, beispielsweise 15 Stunden, bei einer Temperatur von etwa + 4°C;
- K. Zentrifugieren der Suspension und Sammeln der überstehenden Lösung;
- L. Filtration der so erhaltenen Lösung, Ansäuern des so erhaltenen klaren Filtrates, insbesondere auf einen pH-Wert von etwa 5,4, vorzugsweise durch Hinzugabe von Milchsäure, und Filtration des angesäuerten Filtrates;
- M. Pasteurisierung des so erhaltenen Filtrates, insbesondere bei einer Temperatur von etwa 60°C während wenigstens 14 Stunden; und
- N. Zentrifugation der so erhaltenen, pasteurisierten, nicht mehr klaren Lösung und Sammeln der überstehenden Lösung.

2. Extrakt nach Anspruch 1, dadurch erhältlich, dass man das am Ende des Schrittes N erhaltene sterile Filtrat anschliessend noch wenigstens einer Ultrafiltration oder Gel-Filtration aussetzt, um Verbindungen mit vorausbestimmten Molekulargewichten entweder zu entfernen oder zu erhalten.

3. Extrakt nach einem der Ansprüche 1 bis 2, dadurch erhältlich, dass man noch in beliebiger Reihenfolge die folgenden Schritte ausführt:
- O. Ultrafiltration dieses sterilen Filtrates, um Verbindungen mit Molekulargewichten von kleiner als 30'000 zu entfernen und separat zu sammeln; und
- P. Ultrafiltration dieses sterilen Filtrates, um Verbindungen mit Molekulargewichten von grösser als 300'000 zu entfernen und separat zu sammeln.

4. Extrakt nach einem der Ansprüche 1 bis 3, dadurch erhältlich, dass man nach einem oder mehrerer der Schritte L, N, O und P zusätzlich noch eine Sterilisation des jeweiligen Filtrates mittels Filtration durchführt.

5. Extrakt nach einem der Ansprüche 1 bis 4, dadurch erhältlich, dass man nach dem Schritt G und vor dem Schritt H noch eine wässrige, organometallische Lösung hinzugibt, vorzugsweise eine 0,1 %-ige Lösung, beispielsweise eine Kupferacetatlösung, Cu(OAc)₂, insbesondere in einer solchen Menge, dass eine solche Konzentration erhalten wird, welche einer Menge von 1,52 g Cu(OAc)₂ pro kg H₂O entspricht.

6. Extrakt nach einem der Ansprüche 1 bis 5, dadurch erhältlich, dass man nach dem Schritt L und nach einer Sterilisation des Filtrates mittels Filtration und vor dem Schritt M noch eine Inkubation des so erhaltenen sterilen Filtrates ausführt, insbesondere bei einer Temperatur von etwa + 35°C bis etwa + 45°C, insbesondere etwa + 39°C, während 4 bis 6 Tagen.

7. Extrakt nach einem der Ansprüche 1 bis 6, dadurch erhältlich, dass man nach dem Schritt N die erhaltene Lösung ansäuert, beispielsweise bis zu einem pH-Wert von 1,0, vorzugsweise mit Salzsäure, insbesondere mit wässriger Salzsäure,
die so angesäuerte Lösung während einigen Stunden, beispielsweise 15 bis 24 Stunden, inkubiert, und
anschliessend in der so erhaltenen Lösung einen gemessenen pH-Wert im Bereich von 5,0 bis 8,0, vorzugsweise 7,0, einstellt.

8. Verfahren zur Herstellung eines Extraktes mit biologischer und/oder therapeutischer Aktivität, welcher in einem pharmazeutischen und/oder kosmetischen Produkt verwendet werden kann, dadurch gekennzeichnet, dass man die in Anspruch 1 erwähnten Schritte A bis N ausführt.

9. Verfahren nach Anspruch 8, dadurch gekennzeichnet, dass man das am Ende des Schrittes N erhaltene sterile Filtrat anschliessend noch wenigstens einer Ultrafiltration oder Gel-Filtration aussetzt, um Verbindungen mit vorausbestimmten Molekulargewichten entweder zu entfernen oder zu erhalten.

10. Verfahren nach einem der Ansprüche 8 bis 9, dadurch gekennzeichnet, dass man noch in beliebiger Reihenfolge die folgenden Schritte ausführt:
- O. Ultrafiltration dieses sterilen Filtrates, um Verbindungen mit Molekulargewichten von kleiner als 30'000 zu entfernen und separat zu sammeln; und
- P. Ultrafiltration dieses sterilen Filtrates, um Verbindungen mit Molekulargewichten von grösser als 300'000 zu entfernen und separat zu sammeln.

11. Verfahren nach einem der Ansprüche 8 bis 10, dadurch gekennzeichnet, dass man nach einem oder mehrerer der Schritte L, N, O und P zusätzlich noch eine Sterilisation des jeweiligen Filtrates mittels Filtration durchführt.

12. Verfahren nach einem der Ansprüche 8 bis 11, dadurch gekennzeichnet, dass man nach dem Schritt G und vor dem Schritt H noch eine wässrige, organometallische Lösung hinzugibt, vorzugsweise eine 0,1 %-ige Lösung, beispielsweise eine Kupferacetatlösung, Cu(OAc)₂, insbesondere in einer solchen Menge, dass eine solche Konzentration erhalten wird, welche einer Menge von 1,52 g Cu(OAc)₂ pro kg H₂O entspricht.

13. Verfahren nach einem der Ansprüche 8 bis 12, dadurch gekennzeichnet, dass man nach dem Schritt L und nach einer Sterilisation des Filtrates mittels Filtration und vor dem Schritt M noch eine Inkubation des so erhaltenen sterilen Filtrates ausführt, insbesondere bei einer Temperatur von etwa + 35°C bis etwa + 45°C, insbesondere etwa + 39°C, während 4 bis 6 Tagen.

14. Verfahren nach einem der Ansprüche 8 bis 13, dadurch gekennzeichnet, dass man nach dem Schritt N die erhaltene Lösung ansäuert, beispielsweise bis zu einem pH-Wert von 1,0, vorzugsweise mit Salzsäure, insbesondere mit wässriger Salzsäure,
die so angesäuerte Lösung während einigen Stunden, beispielsweise 15 bis 24 Stunden, inkubiert, und
anschliessend in der so erhaltenen Lösung einen gemessenen pH-Wert im Bereich von 5,0 bis 8,0, vorzugsweise 7,0, einstellt.

15. Medikament, insbesondere für die Wiederepithelisierung oder für die Behandlung von Neuropathien, dadurch gekennzeichnet, dass es als wenigstens eine aktive Komponente einen Extrakt nach einem der Ansprüche 1 bis 7 enthält.

16. Medikament nach Anspruch 15, dadurch gekennzeichnet, dass es die Form einer injizierbaren, sterilen Lösung oder alle Formen für lokale Applikationen hat, wie etwa eine Lösung, Salbe, Crème, Gel, Ovuli, Suppositorien, insbesondere eine ophthalmologische Lösung.

17. Verwendung des Extraktes nach einem der Ansprüche 1 bis 7 zur Herstellung eines Medikamentes für die Wiederepithelisierung.

18. Verwendung des Extraktes nach einem der Ansprüche 1 bis 7 zur Herstellung eines Medikamentes für die Behandlung von Neuropathien.

## Patentansprüche (Patentansprüche für folgende(n) Vertragsstaat(en): ES)

1. Verfahren zur Herstellung eines Extraktes mit biologischer und/oder therapeutischer Aktivität, welcher in einem pharmazeutischen und/oder kosmetischen Produkt verwendet werden kann, dadurch erhältlich, dass man die folgenden Schritte ausführt:
- A. Entfernung der Chorion, auch Zottenhaut genannt, der Nabelschnur, Blutgerinnseln und nekrotischem Gewebe von Placentas menschlichen oder tierischen Ursprungs;
- B. Entfernung von Blut von den so erhaltenen Placenta-Geweben unter Einwirkung von Druck, insbesondere durch manuelles Zusammendrücken;
- C. Abtropfen, und gegebenenfalls Abtrocknen, sowie Einfrieren auf eine Temperatur von wenigstens -10°C der so erhaltenen Placenta-Teile;
- D. Zerkleinerung, resp. Pürieren der eingefrorenen Placenta-Teile im gefrorenen Zustand, wobei insbesondere keine Teilchen mit einem Durchmesser von mehr als 5 mm, vorzugsweise 3 mm, vorhanden sind;
- E. Vermischen der so erhaltenen breiigen Masse mit kaltem Wasser, welches gegebenenfalls NaCl enthalten kann;
- F. Rühren der so erhaltenen Suspension während wenigstens 15 Minuten und anschliessendes Stehenlassen bei einer Temperatur von etwa +4°C, beispielsweise während etwa 1 Stunde;
- G. Zugabe von festem Natriumchlorid, um eine physiologische Konzentration, d.h. 8,5 g bis 9,0 g NaCl pro kg H₂O, zu erhalten;
- H. Rühren der erhaltenen Suspension bei einer Temperatur von nicht mehr als + 15°C, insbesondere + 10°C, während einigen Minuten, vorzugsweise etwa 30 Minuten, und anschliessendes Stehenlassen bei einer Temperatur von etwa + 4°C während einigen Stunden, beispielsweise etwa 4 Stunden;
- I. Messen des pH-Wertes und Ansäuern der Suspension, insbesondere auf einen pH-Wert von etwa 5,3, vorzugsweise durch Hinzugabe von Milchsäure;
- J. Lagerung der angesäuerten Suspension während einigen Stunden, beispielsweise 15 Stunden, bei einer Temperatur von etwa + 4°C;
- K. Zentrifugieren der Suspension und Sammeln der überstehenden Lösung;
- L. Filtration der so erhaltenen Lösung, Ansäuern des so erhaltenen klaren Filtrates, insbesondere auf einen pH-Wert von etwa 5,4, vorzugsweise durch Hinzugabe von Milchsäure, und Filtration des angesäuerten Filtrates;
- M. Pasteurisierung des so erhaltenen Filtrates, insbesondere bei einer Temperatur von etwa 60°C während wenigstens 14 Stunden; und
- N. Zentrifugation der so erhaltenen, pasteurisierten, nicht mehr klaren Lösung und Sammeln der überstehenden Lösung.

2. Verfahren zur Herstellung eines Extraktes nach Anspruch 1, dadurch erhältlich, dass man das am Ende des Schrittes N erhaltene sterile Filtrat anschliessend noch wenigstens einer Ultrafiltration oder Gel-Filtration aussetzt, um Verbindungen mit vorausbestimmten Molekulargewichten entweder zu entfernen oder zu erhalten.

3. Verfahren zur Herstellung eines Extraktes nach einem der Ansprüche 1 bis 2, dadurch erhältlich, dass man noch in beliebiger Reihenfolge die folgenden Schritte ausführt:
- O. Ultrafiltration dieses sterilen Filtrates, um Verbindungen mit Molekulargewichten von kleiner als 30'000 zu entfernen und separat zu sammeln; und
- P. Ultrafiltration dieses sterilen Filtrates, um Verbindungen mit Molekulargewichten von grösser als 300'000 zu entfernen und separat zu sammeln.

4. Verfahren zur Herstellung eines Extraktes nach einem der Ansprüche 1 bis 3, dadurch erhältlich, dass man nach einem oder mehrerer der Schritte L, N, O und P zusätzlich noch eine Sterilisation des jeweiligen Filtrates mittels Filtration durchführt.

5. Verfahren zur Herstellung eines Extraktes nach einem der Ansprüche 1 bis 4, dadurch erhältlich, dass man nach dem Schritt G und vor dem Schritt H noch eine wässrige, organometallische Lösung hinzugibt, vorzugsweise eine 0,1 %-ige Lösung, beispielsweise eine Kupferacetatlösung, Cu(OAc)₂, insbesondere in einer solchen Menge, dass eine solche Konzentration erhalten wird, welche einer Menge von 1,52 g Cu(OAc)₂ pro kg H₂O entspricht.

6. Verfahren zur Herstellung eines Extraktes nach einem der Ansprüche 1 bis 5, dadurch erhältlich, dass man nach dem Schritt L und nach einer Sterilisation des Filtrates mittels Filtration und vor dem Schritt M noch eine Inkubation des so erhaltenen sterilen Filtrates ausführt, insbesondere bei einer Temperatur von etwa + 35°C bis etwa + 45°C, insbesondere etwa + 39°C, während 4 bis 6 Tagen.

7. Verfahren zur Herstellung eines Extraktes nach einem der Ansprüche 1 bis 6, dadurch erhältlich, dass man nach dem Schritt N die erhaltene Lösung ansäuert, beispielsweise bis zu einem pH-Wert von 1,0, vorzugsweise mit Salzsäure, insbesondere mit wässriger Salzsäure,
die so angesäuerte Lösung während einigen Stunden, beispielsweise 15 bis 24 Stunden, inkubiert, und
anschliessend in der so erhaltenen Lösung einen gemessenen pH-Wert im Bereich von 5,0 bis 8,0, vorzugsweise 7,0, einstellt.

8. Verfahren zur Herstellung des Extraktes nach den vorhergehenden Ansprüchen für die Benutzung als Medikament, insbesondere für die Wiederepithelisierung oder für die Behandlung von Neuropathien, dadurch gekennzeichnet, dass es als wenigstens eine aktive Komponente einen Extrakt nach einem der Ansprüche 1 bis 7 enthält.

9. Verfahren zur Herstellung des Medikamentes nach Anspruch 8, dadurch gekennzeichnet, dass es die Form einer injizierbaren, sterilen Lösung oder alle Formen für lokale Applikationen hat, wie etwa eine Lösung, Salbe, Crème, Gel, Ovuli, Suppositorien, insbesondere eine ophthalmologische Lösung.

10. Verfahren zur Verwendung des Extraktes nach einem der Ansprüche 1 bis 7 zur Herstellung eines Medikamentes für die Wiederepithelisierung.

11. Verfahren zur Verwendung des Extraktes nach einem der Ansprüche 1 bis 7 zur Herstellung eines Medikamentes für die Behandlung von Neuropathien.

## Claims (Claims for the following Contracting State(s): AT, BE, CH, DE, DK, FR, GB, GR, IT, LI, LU, NL, PT, SE, IE)

1. An extract having biological and/or therapeutical activity, which may be used in a pharmaceutical and/or cosmetical product, obtainable in that the following steps are carried out:
- A. removing the chorion, the cort, blood clots and necrotic tissues of placentas of human or animal origin;
- B. removing of blood from the so obtained placenta tissues under the influence of pressure, especially by manual compressing;
- C. draining, and occasionally wiping dry, as well as freezing in to a temperature of at least - 10°C of the so obtained placenta parts;
- D. reducing to small pieces and mashing respectively of the frozen placenta parts in the frozen state, whereby especially no particles are present having a diameter of more than 5 mm, preferably 3 mm;
- E. mixing of the so obtained mashed mass with cold water which occasionally may contain NaCl;
- F. stirring of the so obtained suspension during at least 15 minutes and following standing at a temperature of about + 4°C, for example during about one hour;
- G. adding of solid sodium chloride, in order to obtain a physiological concentration, i.e. 8.5 g to 9.0 g NaCl per kg H₂O;
- H. stirring of the obtained suspension at a temperature of not more than +15°C, especially + 10°C, during a few minutes, preferably about 30 minutes, and following standing at a temperature of about + 4°C during a few hours, for example about 4 hours;
- I. measuring of the pH-value and acidifying of the suspension, especially to a pH-value of about 5.3, preferably by the addition of lactic acid;
- J. storing of the acidified suspension during a few hours, for example 15 hours, at a temperature of about + 4°C;
- K. centrifugating of the suspension and collecting of the supernatant solution;
- L. filtrating of the so obtained solution, acidifying of the so obtained clear filtrate, especially to a pH-value of about 5.4, preferably by the addition of lactic acid, and filtrating the acidified filtrate;
- M. pasteurizing of the so obtained filtrate, especially at a temperature of about 60°C during at least 14 hours; and
- N. centrifugating of the so obtained pasteurized, no more clear solution and collecting of the supernatant solution.

2. The extract according to claim 1, obtainable in that the steril filtrate obtained at the end of step N is then subjected to at least one ultrafiltration or gel filtration, in order to either remove or to obtain compounds having predetermined molecular weights.

3. The extract according to one of claims 1 to 2, obtainable in that in any sequence the following steps are carried out:
- O. ultrafiltrating of this steril filtrate, in order to remove and to collect separately compounds having molecular weights of less than 30'000; and
- P. ultrafiltrating of this steril filtrate, in order to remove and to collect separately compounds having molecular weights of more than 300'000.

4. The extract according to one of claims 1 to 3, obtainable in that after one or more of the steps L, N, O and P additionally a sterilization of the respective filtrate by means of filtration is carried out.

5. The extract according to one of claims 1 to 4, obtainable in that after step G and before step H an aqueous, organo metallic solution is added, preferably a 0.1 % solution, for example a copper (II) acetate solution, Cu(OAc)₂, especially in such an amount that such a concentration is obtained, which corresponds to an amount of 1.52 g Cu(OAc)₂ per kg H₂O.

6. The extract according to one of claims 1 to 5, obtainable in that after step L and after a sterilization of the filtrate by means of a filtration and before step M an incubation of the so obtained steril filtrate is carried out, especially at a temperature from about + 35°C to about + 45°C, especially about + 39°C, during 4 to 6 days.

7. The extract according to one of claims 1 to 6, obtainable in that after step N the obtained solution is acidified, for example up to a pH-value of 1.0, preferably with hydrochloric acid, especially with aqueous hydrochloric acid,
the so acidified solution is incubated during some hours, for example 15 to 24 hours, and
then is adjusted in the so obtained solution a measured pH-value in the range from 5.0 to 8.0, preferably 7.0.

8. A process for the preparation of an extract having biological and/or therapeutical acitvity, which may be used in a pharmaceutical and/or cosmetical product, characterized in that the steps A to N as mentioned in claim 1 are carried out.

9. The process according to claim 8, characterized in that the steril filtrate obtained at the end of step N is then subjected to at least one ultrafiltration or gel filtration, in order to either remove or to obtain compounds having predetermined molecular weights.

10. The process according to one of claims 8 to 9, characterized in that in any sequence the following steps are carried out:
- O. ultrafiltrating of this steril filtrate, in order to remove and to collect separately compounds having molecular weights of less than 30'000; and
- P. ultrafiltrating of this steril filtrate, in order to remove and to collect separately compounds having molecular weights of more than 300'000.

11. The process according to one of claims 8 to 10, characterized in that after one or more of the steps L, N, O and P additionally a sterilization of the respective filtrate by means of filtration is carried out.

12. The process according to one of claims 8 to 11, characterized in that after step G and before step H an aqueous, organo metallic solution is added, preferably a 0.1 % solution, for example a copper (II) acetate solution, Cu(OAc)₂, especially in such an amount that such a concentration is obtained, which corresponds to an amount of 1.52 g Cu(OAc)₂ per kg H₂O.

13. The process according to one of claims 8 to 12, characterized in that after step L and after a sterilization of the filtrate by means of a filtration and before step M an incubation of the so obtained steril filtrate is carried out, especially at a temperature from about + 35°C to about + 45°C, especially about + 39°C, during 4 to 6 days.

14. The process according to one of claims 8 to 13, characterized in that after step N the obtained solution is acidified, for example up to a pH-value of 1.0, preferably with hydrochloric acid, especially with aqueous hydrochloric acid,
the so acidified solution is incubated during some hours, for example 15 to 24 hours, and
then is adjusted in the so obtained solution a measured pH-value in the range from 5.0 to 8.0, preferably 7.0.

15. A medicament, especially for the reepithelization or for the treatment of neuropathias, characterized in that it contains as at least one active component an extract according to one of claims 1 to 7.

16. The medicament according to claim 15, characterized in that it has the form of an injectable, steril solution or all forms for local applications, such as a solution, ointment, cream, gel, ovuli, suppositories, especially an ophthalmological solution.

17. Use of the extract according to one of claims 1 to 7 for the preparation of a medicament for the reepithelization.

18. Use of the extract according to one of claims 1 to 7 for the preparation of a medicament for the treatment of neuropathias.

## Claims (Claims for the following Contracting State(s): ES)

1. A process for the preparation of an extract having biological and/or therapeutical activity, which may be used in a pharmaceutical and/or cosmetical product, obtainable in that the following steps are carried out:
- A. removing the chorion, the cort, blood clots and necrotic tissues of Placentas of human or animal origin;
- B. removing of blood from the so obtained placenta tissues under the influence of pressure, especially by manual compressing;
- C. draining, and occasionally wiping dry, as well as freezing in to a temperature of at least - 10°C of the so obtained placenta parts;
- D. reducing to small pieces and mashing respectively of the frozen placenta parts in the frozen state, whereby especially no particles are present having a diameter of more than 5 mm, preferably 3 mm;
- E. mixing of the so obtained mashed mass with cold water which occasionally may contain NaCl;
- F. stirring of the so obtained suspension during at least 15 minutes and following standing at a temperature of about + 4°C, for example during about one hour;
- G. adding of solid sodium chloride, in order to obtain a physiological concentration, i.e. 8.5 g to 9.0 g NaCl per kg H₂O;
- H. stirring of the obtained suspension at a temperature of not more than +15°C, especially + 10°C, during a few minutes, preferably about 30 minutes, and following standing at a temperature of about + 4°C during a few hours, for example about 4 hours;
- I. measuring of the pH-value and acidifying of the suspension, especially to a pH-value of about 5.3, preferably by the addition of lactic acid;
- J. storing of the acidified suspension during a few hours, for example 15 hours, at a temperature of about + 4°C;
- K. centrifugating of the suspension and collecting of the supernatant solution;
- L. filtrating of the so obtained solution, acidifying of the so obtained clear filtrate, especially to a pH-value of about 5.4, preferably by the addition of lactic acid, and filtrating the acidified filtrate;
- M. pasteurizing of the so obtained filtrate, especially at a temperature of about 60°C during at least 14 hours; and
- N. centrifugating of the so obtained pasteurized, no more clear solution and collecting of the supernatant solution.

2. The process for the preparation of an extract according to claim 1, obtainable in that the steril filtrate obtained at the end of step N is then subjected to at least one ultrafiltration or gel filtration, in order to either remove or to obtain compounds having predetermined molecular weights.

3. The process for the preparation of an extract according to one of claims 1 to 2, obtainable in that in any sequence the following steps are carried out:
- O. ultrafiltrating of this steril filtrate, in order to remove and to collect separately compounds having molecular weights of less than 30'000; and
- P. ultrafiltrating of this steril filtrate, in order to remove and to collect separately compounds having molecular weights of more than 300'000.

4. The process for the preparation of an extract according to one of claims 1 to 3, obtainable in that after one or more of the steps L, N, O and P additionally a sterilization of the respective filtrate by means of filtration is carried out.

5. The process for the preparation of an extract according to one of claims 1 to 4, obtainable in that after step G and before step H an aqueous, organo metallic solution is added, preferably a 0.1 % solution, for example a copper (II) acetate solution, Cu(OAc)₂, especially in such an amount that such a concentration is obtained, which corresponds to an amount of 1.52 g Cu(OAc)₂ per kg H₂O.

6. The process for the preparation of an extract according to one of claims 1 to 5, obtainable in that after step L and after a sterilization of the filtrate by means of a filtration and before step M an incubation of the so obtained steril filtrate is carried out, especially at a temperature from about + 35°C to about + 45°C, especially about + 39°C, during 4 to 6 days.

7. The process for the preparation of an extract according to one of claims 1 to 6, obtainable in that after step N the obtained solution is acidified, for example up to a pH-value of 1.0, preferably with hydrochloric acid, especially with aqueous hydrochloric acid,
the so acidified solution is incubated during some hours, for example 15 to 24 hours, and
then is adjusted in the so obtained solution a measured pH-value in the range from 5.0 to 8.0, preferably 7.0.

8. A process for the preparation of the extract according to the proceeding claims for the use as medicament, especially for the reepithelization or for the treatment of neuropathias, characterized in that it contains as at least one active component an extract according to one of claims 1 to 7.

9. The process for the preparation of the medicament according to claim 8, characterized in that it has the form of an injectable, steril solution or all forms for local applications, such as a solution, ointment, cream, gel, ovuli, suppositories, especially an ophthalmological solution.

10. A process for the use of the extract according to one of claims 1 to 7 for the preparation of a medicament for the reepithelization.

11. A process for the use of the extract according to one of claims 1 to 7 for the preparation of a medicament for the treatment of neuropathias.

## Revendications (Revendications pour l'(les) Etat(s) contractant(s) suivant(s): AT, BE, CH, DE, DK, FR, GB, GR, IT, LI, LU, NL, PT, SE, IE)

1. Extrait possédant une activité biologique et/ou thérapeutique et se prêtant à un usage dans un produit pharmaceutique et/ou cosmétique, qui peut être obtenu en exécutant les étapes suivantes:
- A. Elimination du chorion, du cordon ombilical, des caillots sanguins et des tissus nécrotiques de placentas d'origine humaine ou animale;
- B. Elimination du sang des tissus placentaires obtenus de la sorte en exerçant une pression, en particulier par compression manuelle;
- C. Egouttage et, facultativement, séchage ainsi que congélation des parties de placenta obtenues de la sorte en abaissant la température à -10 °C au moins;
- D. Broyage ou mise en purée, à l'état congelé, des parties de placenta congelées en veillant en particulier à ce qu'il n'y ait pas de fragments d'un diamètre supérieur à 5 mm, de préférence à 3 mm;
- E. Mélange de la masse pâteuse obtenue de la sorte, avec de l'eau froide pouvant contenir facultativement du NaCl;
- F. Agitation de la suspension qui a été obtenue de la sorte pendant au moins 15 minutes, puis décantation à une température d'environ +4 °C, par exemple pendant 1 heure environ;
- G. Addition de chlorure de sodium solide de façon à obtenir une concentration physiologique, soit 8,5 g à 9,0 g de NaCl par kg de H₂O;
- H. Agitation de la suspension obtenue à une température ne dépassant pas +15 °C, en particulier +10 °C, pendant quelques minutes, de préférence pendant 30 minutes environ, puis décantation à une température d'environ +4 °C pendant quelques heures, par exemple pendant 4 heures environ;
- I. Mesure du pH et acidification de la suspension, en particulier pour l'amener à un pH d'environ 5,3, de préférence par addition d'acide lactique;
- J. Stockage de la suspension acidifiée pendant quelques heures, par exemple pendant 15 heures, à une température d'environ +4 °C;
- K. Centrifugation de la suspension et collecte de la solution surnageante;
- L. Filtration de la solution obtenue de la sorte, acidification du filtrat clair obtenu de la sorte, en particulier pour l'amener à un pH d'environ 5,4, de préférence par addition d'acide lactique, et filtration du filtrat acidifié;
- M. Pasteurisation du filtrat obtenu de la sorte, en particulier à une température d'environ 60 °C pendant au moins 14 heures; et
- N. Centrifugation de la solution pasteurisée, trouble à présent, obtenue de la sorte, et collecte de la solution surnageante.

2. Extrait selon la revendication 1, qui peut être obtenu en soumettant le filtrat stérile obtenu à l'issue de l'étape N à au moins encore une ultrafiltration ou filtration sur gel dans le but soit d'éliminer soit de recueillir des composés de masses moléculaires prédéfinies.

3. Extrait selon l'une des revendications 1 à 2, qui peut être obtenu en exécutant dans un ordre arbitraire les étapes suivantes:
- O. Ultrafiltration de ce filtrat stérile afin d'en séparer des composés de masses moléculaires inférieures à 30'000 et de les recueillir à part; et
- P. Ultrafiltration de ce filtrat stérile afin d'en séparer des composés de masses moléculaires supérieures à 300'000 et de les recueillir à part.

4. Extrait selon l'une des revendications 1 à 3, qui peut être obtenu en procédant encore, après une ou plusieurs des étapes L, N, O et P, à une stérilisation supplémentaire de chaque filtrat par filtration.

5. Extrait selon l'une des revendications 1 à 4, qui peut être obtenu en ajoutant encore, après l'étape G et avant l'étape H, une solution organométallique aqueuse, de préférence à 0,1 %, par exemple une solution d'acétate de cuivre - Cu(OAc)₂ -, en particulier dans une quantité telle que la concentration obtenue corresponde à une quantité de 1,52 g de Cu(OAc)₂ par kg de H₂O.

6. Extrait selon l'une des revendications 1 à 5, qui peut être obtenu en procédant en plus, après l'étape L, après une stérilisation du filtrat par filtration et avant l'étape M, à une incubation du filtrat stérile obtenu de la sorte, en particulier à une température allant de +35 °C environ à +45 °C environ, en particulier égale à +39 °C environ, pendant 4 à 6 jours.

7. Extrait selon l'une des revendications 1 à 6, qui peut être obtenu en acidifiant, après l'étape N, la solution obtenue, par exemple de façon à porter son pH à 1,0, de préférence au moyen d'acide chlorhydrique, en particulier au moyen d'acide chlorhydrique aqueux,
en incubant la solution acidifiée de la sorte pendant quelques heures, par exemple pendant 15 à 24 heures, et
en amenant ensuite le pH de la solution obtenue de la sorte à une valeur appropriée située dans la plage allant de 5,0 à 8,0, de préférence 7,0.

8. Procédé de préparation d'un extrait possédant une activité biologique et/ou thérapeutique et se prêtant à un usage dans un produit pharmaceutique et/ou cosmétique, caractérisé en ce que les étapes A à N mentionnées à la revendication 1 sont exécutées.

9. Procédé selon la revendication 8, caractérisé en ce que le filtrat stérile obtenu à l'issue de l'étape N est ensuite soumis à au moins encore une ultrafiltration ou filtration sur gel afin d'éliminer ou de recueillir des composés de masses moléculaires prédéfinies.

10. Procédé selon l'une des revendications 8 à 9, caractérisé en ce que les étapes ci-après sont exécutées dans un ordre arbitraire:
- O. Ultrafiltration de ce filtrat stérile pour séparer les composés de masses moléculaires inférieures à 30'000 et les recueillir à part; et
- P. Ultrafiltration de ce filtrat stérile pour séparer les composés de masses moléculaires supérieures à 300'000 et les recueillir à part.

11. Procédé selon l'une des revendications 8 à 10, caractérisé en ce qu'après une ou plusieurs des étapes L, N, O et P, une stérilisation supplémentaire de chaque filtrat obtenu est encore réalisée par filtration.

12. Procédé selon l'une des revendications 8 à 11, caractérisé en ce qu'après l'étape G et avant l'étape H, une solution organométallique aqueuse, de préférence à 0,1 %, est ajoutée, par exemple une solution d'acétate de cuivre - Cu(OAc)₂ -, en particulier dans une quantité telle que la concentration obtenue corresponde à une quantité de 1,52 g de Cu(OAc)₂ par kg de H₂O.

13. Procédé selon l'une des revendications 8 à 12, caractérisé en ce qu'après l'étape L, après une stérilisation du filtrat par filtration et avant l'étape M, le filtrat obtenu de la sorte est soumis à une incubation, en particulier à une température d'environ +35 °C à environ +45 °C, en particulier d'environ +39 °C, pendant 4 à 6 jours.

14. Procédé selon l'une des revendications 8 à 13, caractérisé en ce qu'après l'étape N, la solution obtenue est acidifiée, par exemple de manière à porter son pH à 1,0, de préférence avec de l'acide chlorhydrique, en particulier avec de l'acide chlorhydrique aqueux,
en ce que la solution ainsi acidifiée est incubée pendant quelques heures, par exemple pendant 15 à 24 heures, et
en ce que le pH de la solution obtenue de la sorte est ensuite porté à une valeur appropriée située dans une plage allant de 5,0 à 8,0, de préférence 7,0.

15. Médicament, en particulier pour la ré-épithélisation ou le traitement des neuropathies, caractérisé en ce qu'au moins l'un de ses composants actifs est un extrait selon l'une des revendications 1 à 7.

16. Médicament selon la revendication 15, caractérisé en ce qu'il a la forme d'une solution stérile injectable ou de tous les types d'application locale tels qu'une solution, une pommade, une crème, un gel, des ovules, des suppositoires, en particulier une solution ophtalmique.

17. Mise en oeuvre de l'extrait selon l'une des revendications 1 à 7 pour préparer un médicament destiné à la ré-épithélisation.

18. Mise en oeuvre de l'extrait selon l'une des revendications 1 à 7 pour préparer un médicament destiné au traitement des neuropathies.

## Revendications (Revendications pour l'(les) Etat(s) contractant(s) suivant(s): ES)

1. Procédé de préparation d'un extrait possédant une activité biologique et/ou thérapeutique et se prêtant à un usage dans un produit pharmaceutique et/ou cosmétique, qui peut être obtenu en exécutant les étapes suivantes:
- A. Elimination du chorion, du cordon ombilical, des caillots sanguins et des tissus nécrotiques de placentas d'origine humaine ou animale;
- B. Elimination du sang des tissus placentaires obtenus de la sorte en exerçant une pression, en particulier par compression manuelle;
- C. Egouttage et, facultativement, séchage ainsi que congélation des parties de placenta obtenues de la sorte en abaissant la température à -10 °C au moins;
- D. Broyage ou mise en purée, à l'état congelé, des parties de placenta congelées en veillant en particulier à ce qu'il n'y ait pas de fragments d'un diamètre supérieur à 5 mm, de préférence à 3 mm;
- E. Mélange de la masse pâteuse obtenue de la sorte avec de l'eau froide pouvant contenir facultativement du NaCl;
- F. Agitation, pendant au moins 15 minutes, de la suspension obtenue de la sorte, puis décantation à une température d'environ +4 °C, par exemple pendant 1 heure environ;
- G. Addition de chlorure de sodium solide afin d'obtenir une concentration physiologique, soit 8,5 g à 9,0 g de NaCl par kg de H₂O;
- H. Agitation de la suspension obtenue à une température ne dépassant pas +15 °C, en particulier +10 °C, pendant quelques minutes, de préférence pendant 30 minutes environ, puis décantation de la suspension à une température voisine de +4 °C pendant quelques heures, par exemple pendant 4 heures environ;
- I. Mesure du pH et acidification de la suspension, en particulier pour l'amener a un pH d'environ 5,3, de préférence par addition d'acide lactique;
- J. Stockage de la suspension acidifiée pendant quelques heures, par exemple pendant 15 heures, à une température d'environ +4 °C;
- K. Centrifugation de la suspension et collecte de la solution surnageante;
- L. Filtration de la solution obtenue de la sorte, acidification du filtrat clair obtenu de la sorte, en particulier pour l'amener à un pH d'environ 5,4, de préférence par addition d'acide lactique, et filtration du filtrat acidifié;
- M. Pasteurisation du filtrat obtenu de la sorte, en particulier à une température d'environ 60 °C pendant au moins 14 heures; et
- N. Centrifugation de la solution pasteurisée, trouble à présent, qui a été obtenue de la sorte, et collecte de la solution surnageante.

2. Procédé de préparation d'un extrait selon la revendication 1, qui peut être obtenu en soumettant ensuite le filtrat stérile obtenu à l'issue de l'étape N à au moins encore une ultrafiltration ou filtration sur gel dans le but soit d'éliminer soit de recueillir des composés de masses moléculaires prédéfinies.

3. Procédé de préparation d'un extrait selon l'une des revendications 1 à 2, qui peut être obtenu en exécutant dans un ordre arbitraire les étapes suivantes:
- O. Ultrafiltration de ce filtrat stérile afin d'en séparer des composés de masses moléculaires inférieures à 30'000 et de les recueillir séparément; et
- P. Ultrafiltration de ce filtrat stérile afin d'en séparer des composés de masses moléculaires supérieures à 300'000 et de les recueillir séparément.

4. Procédé de préparation d'un extrait selon l'une des revendications 1 à 3, qui peut être obtenu en réalisant encore, après une ou plusieurs des étapes L, N, O et P, une stérilisation supplémentaire de chaque filtrat par filtration.

5. Procédé de préparation d'un extrait selon l'une des revendications 1 à 4, qui peut être obtenu en ajoutant encore, après l'étape G et avant l'étape H, une solution organométallique aqueuse, de préférence à 0,1 %, par exemple une solution d'acétate de cuivre - Cu(OAc)₂ -, en particulier dans une quantité telle que la concentration obtenue corresponde à une quantité de 1,52 g de Cu(OAc)₂ par kg de H₂O.

6. Procédé de préparation d'un extrait selon l'une des revendications 1 à 5, qui peut être obtenu en procédant en plus, après l'étape L, après une stérilisation du filtrat par filtration et avant l'étape M, à une incubation du filtrat stérile obtenu de la sorte, en particulier à une température allant de +35 °C environ à +45 °C environ, en particulier de +39 °C environ, pendant 4 à 6 jours.

7. Procédé de préparation d'un extrait selon l'une des revendications 1 à 6 qui peut être obtenu en acidifiant la solution obtenue après l'étape N, par exemple de façon à porter son pH à 1,0, de préférence au moyen d'acide chlorhydrique, en particulier au moyen d'acide chlorhydrique aqueux,
en incubant la solution acidifiée de la sorte pendant quelques heures, par exemple pendant 15 à 24 heures, et
en amenant ensuite le pH de la solution obtenue de la sorte à une valeur appropriée située dans la plage allant de 5,0 à 8,0, de préférence 7,0.

8. Procédé de préparation d'un extrait selon les revendications précédentes, destiné à être utilisé comme médicament, en particulier pour la ré-épithélisation ou le traitement des neuropathies, caractérisé en ce qu'au moins l'un de ses principes actifs est un extrait selon l'une des revendications 1 à 7.

9. Procédé de préparation du médicament selon la revendication 8, caractérisé en ce qu'il a la forme d'une solution stérile injectable ou de tous les types d'application locale tels qu'une solution, une pommade, une crème, un gel, des ovules, des suppositoires, en particulier une solution ophtalmique.

10. Procédé de mise en oeuvre de l'extrait selon l'une des revendications 1 à 7 pour préparer un médicament destiné à la ré-épithélisation.

11. Procédé de mise en oeuvre de l'extrait selon l'une des revendications 1 à 7 pour préparer un médicament destiné au traitement des neuropathies.
